# EUROPEAN PATENT SPECIFICATION

(11) **EP 4 248 927 B1**
(45) Date of publication and mention of the grant of the patent: **03.12.2025**
(21) Application number: 21915092.7
(22) Date of filing: 15.12.2021
(51) Int. Cl.: A61F 13/53, C08F 8/12, A61L 15/60, A61F 13/15

(54) **COMPOSITE ABSORBENT AND SANITARY MATERIAL**
ZUSAMMENGESETZTES ABSORBIERENDES UND SANITÄRES MATERIAL
ABSORBANT COMPOSITE ET MATÉRIAU SANITAIRE

(30) Priority: 29.12.2020 JP 2020219818
(43) Date of publication of application: 27.09.2023
(73) Proprietor: Unicharm Corporation, Shikokuchuo-shi, Ehime 799-0111 (JP)
(72) Inventor: KIKUCHI, Kyo, Kanonji-shi, Kagawa 769-1602 (JP); KINOSHITA, Akie, Kanonji-shi, Kagawa 769-1602 (JP)
(74) Representative: Dolleymores
(86) International application number: PCT/JP2021/046266
(87) International publication number: WO 2022/145231

(56) References cited:
- EP-A1- 0 993 310
- EP-B1- 0 993 310
- WO-A1-2019/110787
- JP-A- 2002 505 702
- JP-A- 2011 500 874
- JP-A- 2017 164 356
- JP-A- H0 670 953
- JP-A- H08 504 474
- US-A1- 2004 181 199
- US-A1- 2007 202 766
- US-A1- 2020 383 846

## Description

### FIELD

The present invention relates to a composite absorbent body and a sanitary product having the same.

### BACKGROUND

In absorbent articles such as disposable diapers and sanitary napkins, those including a porous material such as a sponge material as an absorbent body are known. For example, Patent Literature 1 discloses an absorbent article containing a polymeric foam material formed of a hydrophilic flexible structure of interconnected open cells. US 2020/383846 A1 discloses an absorbent article and a method of manufacturing the absorbent article.

### [CITATION LIST]

### [PATENT LITERATURE]

[Patent Literature 1]
Japanese Patent No. 3231320

### SUMMARY

### [TECHNICAL PROBLEM]

According to the studies conducted by the inventors of the present invention, in a porous material such as a polymeric foam material of Patent Literature 1, there are many pores having a relatively small pore radius. However, in a porous structure, water tends to be drawn into pores having a relatively large pore radius. Therefore, in the above-described porous material having a large number of pores having a small pore diameter, there is a risk that, during water absorption, no water enters pores having a small pore diameter, and the water absorption amount decreases relative with the pore volume.

The present invention was made in view of the above problem, and an object of the present invention is to provide a composite absorbent body for a sanitary product, capable of suppressing a decrease in water absorption amount relative to a pore volume and having excellent absorption performance, and a sanitary product having the same.

### [MEANS FOR SOLVING PROBLEMS]

One aspect (Aspect 1) of the present invention is defined in claim 1.

In the composite absorbent body according to the present aspect, in the polymer absorbent, the ratio of the pore volume of the pore having a pore radius of 1 µm or more is 90% or more of the pore volume of the pore. Therefore, during water absorption, even when water is less likely to enter the pores having a relatively small pore radius such as a pore radius of less than 1 µm, or water cannot enter the pores, water can enter the pores having a pore radius of 1 µm or more, which makes it possible to secure a sufficient water absorption amount. Accordingly, a decrease in the water absorption amount relative to the pore volume can be suppressed, and excellent absorption performance can be obtained.

In addition, another aspect (Aspect 2) of the present invention is:
the composite absorbent body according to Aspect 1, wherein
in the polymer absorbent, a ratio of a pore volume of the pore having the pore radius of 0.005 µm or less is less than 10% of the pore volume of the pore.

In the composite absorbent body according to the present aspect, the ratio of the pore volume of the pore having a very small pore radius, such as a pore radius of 0.005 µm or less, and difficult to absorb water is very small, and the ratio of the pore volume of the pore having a large pore radius, such as a pore radius of 1 µm or more, and capable of absorbing water is large. Accordingly, the pores of the polymer absorbent can be effectively utilized for water absorption, and a sufficient water absorption amount can be secured.

Still another aspect (Aspect 3) of the present invention is:
the composite absorbent body according to Aspect 1 or 2, wherein
in the polymer absorbent, a pore radius at a maximum value of a pore volume is 500 µm or less.

In the composite absorbent body according to the present aspect, when the pore radius at the maximum value of the pore volume is set to 500 µm or less, during water absorption, the collapse of the structure of the continuous skeleton of the polymer absorbent can be suppressed, and an excellent absorption rate can be easily obtained, which makes it possible to stably secure a sufficient water absorption amount (in a case where the pore radius at the maximum value of the pore volume is 500 µm or more, there is a risk that the structure of the continuous skeleton cannot be maintained and collapse during water absorption).

Still another aspect (Aspect 4) of the present invention is:
the composite absorbent body according to any one of Aspects 1 to 3, wherein
in the polymer absorbent, a variation coefficient of a pore distribution in the pores having a pore radius of 1 µm or more is 1.4 or less.

In the composite absorbent body according to the present aspect, since the variation coefficient of the pore distribution is 1.4 or less, the variation of the pore radius with respect to the average value of the pore radii is small, and the peak indicated by the pore distribution becomes sharp in the vicinity of the average value of the pore radii. Therefore, the polymer absorbent can absorb water substantially uniformly from the entire surface and in all directions. Accordingly, the pores of the polymer absorbent can be effectively utilized for water absorption, and a sufficient water absorption amount can be secured.

Still another aspect (Aspect 5) of the present invention is:
the composite absorbent body according to any one of Aspects 1 to 3, wherein
in the polymer absorbent, a variation coefficient of a pore distribution in the pores having a pore radius of 1 µm or more is 1.4 or less.

In the composite absorbent body according to the present aspect, since the variation coefficient of the pore distribution is more than 1.4, the variation of the pore radius with respect to the average value of the pore radii is large, and the peak indicated by the pore distribution becomes broad in the vicinity of the average value of the pore radii. That is, the polymer absorbent has pores having a small pore radius and pores having a large pore radius. Therefore, in the pores having a small pore radius, a capillary force easily acts, and thus the water absorption rate is easily increased. In the pores having a large pore radius, the water absorption volume is easily increased. Therefore, due to two synergistic effects, a polymer absorbent can instantaneously absorb a large amount of water inside the pores.

Still another aspect (Aspect 6) of the present invention is:
the composite absorbent body according to Aspect 5, wherein
in the polymer absorbent, a portion on a side with a larger pore radius is broader than a portion on a side with a smaller pore radius with respect to a pore radius corresponding to a local maximum value of the pore volume in a curved line showing a pore distribution.

The composite absorbent body according to the present aspect has a configuration in which the polymer absorbent has the above-described configuration, that is, a configuration in which there are more pores having a larger pore diameter than pores having a smaller pore radius. Since there are a large number of pores having a large pore radius, the water absorption volume is more easily increased, and a larger amount of water can be absorbed inside the pores.

Still another aspect (Aspect 7) of the present invention is:
the composite absorbent body according to Aspect 5, wherein
in the polymer absorbent, a portion on a side with a smaller pore radius is broader than a portion on a side with a larger pore radius with respect to a pore radius corresponding to a local maximum value of the pore volume in a curved line showing a pore distribution.

The composite absorbent body according to the present aspect has a configuration in which the polymer absorbent has the above-described configuration, that is, a configuration in which there are more pores having a smaller pore diameter than pores having a larger pore radius. The large number of pores having a small pore radius makes the capillary force more likely to work, the water absorption rate is more easily increased, and water can be more instantaneously absorbed inside the pores.

Still another aspect (Aspect 8) of the present invention is:
the composite absorbent body according to any one of Aspects 1 to 3, wherein
in the polymer absorbent, at least two local maximum values of the pore volume are present in a curved line showing a pore distribution.

In the composite absorbent body according to the present aspect, the polymer absorbent has the above-described configuration, that is, a configuration in which there are pores having a predetermined small pore radius and a pore radius in the vicinity thereof, and pores having a predetermined large pore radius and a pore radius in the vicinity thereof. Therefore, in the pores having a relatively small pore radius, a capillary force easily acts, and thus the water absorption rate is easily increased. In the pores having a relatively large pore radius, the water absorption volume is easily increased. Therefore, due to two synergistic effects, a polymer absorbent can instantaneously absorb a large amount of water inside the pores.

Still another aspect (Aspect 9) of the present invention is:
the composite absorbent body according to Aspect 8, wherein
in the polymer absorbent, of the two local maximum values of the pore volume in the curved line showing the pore distribution, a relatively small local maximum value of a pore radius is larger than a relatively large local maximum value of the pore radius.

In the composite absorbent body according to the present aspect, the polymer absorbent has the above-described configuration, that is, there are more pores having a smaller pore diameter than pores having a larger pore radius. The large number of pores having a small pore radius makes the capillary force more likely to work, the water absorption rate is more easily increased, and water can be more instantaneously absorbed inside the pores.

Still another aspect (Aspect 10) of the present invention is:
the composite absorbent body according to Aspect 8, wherein
in the polymer absorbent, of the two local maximum values of the pore volume in the curved line showing the pore distribution, a relatively small local maximum value of a pore radius is smaller than a relatively large local maximum value of the pore radius.

In the composite absorbent body according to the present aspect, the polymer absorbent has the above-described configuration, that is, there are more pores having a larger pore diameter than pores having a smaller pore radius. Since there are a large number of pores having a large pore radius, the water absorption volume is more easily increased, and a larger amount of water can be absorbed inside the pores.

Still another aspect (Aspect 11) of the present invention is:
the composite absorbent body according to any one of Aspects 1 to 10, wherein
the polymer absorbent has a total pore volume of 0.9 mL/g or more.

In the composite absorbent body according to the present aspect, in the polymer absorbent, the total pore volume is 0.9 mL/g or more. Accordingly, a sufficient pore volume can be secured in the polymer absorbent, and therefore a sufficient water absorption amount can be secured. Further, the spaces (pores) for taking in the liquid to be absorbed (bodily fluid) in the porous body can be made less likely to collapse during absorption, and the water absorption amount and the water absorption rate can be made less likely to decrease.

Still another aspect (Aspect 12) of the present invention is:
the composite absorbent body according to any one of Aspects 1 to 11, wherein
the polymer absorbent has a bulk density of 0.07 to 0.6 g/cm³.

In the composite absorbent body according to the present aspect, since the bulk density in the polymer absorbent is 0.07 to 0.6 g/cm³, the water absorption rate (DW) performance can be set to 6 mL/30 sec or higher. That is, since the water absorption rate is high, the polymer absorbent can more instantaneously absorb water inside the pores.

Still another aspect (Aspect 13) of the present invention is:
the composite absorbent body according to any one of Aspects 1 to 12, wherein
the polymer absorbent is a monolithic absorbent.

In the composite absorbent body according to the present aspect, since the polymer absorbent is a monolithic absorbent, water can be rapidly absorbed.

Still another aspect (Aspect 14) of the present invention is:
the composite absorbent body according to any one of Aspects 1 to 13, wherein
the polymer absorbent is a hydrolysate of a crosslinked polymer of a (meth)acrylic acid ester and a compound containing two or more vinyl groups in one molecule, and contains at least one or more -COONa groups.

In the composite absorbent body according to the present aspect, since the polymer absorbent has the above-described specific configuration, the hydrophilic continuous skeleton easily extends, and the continuous pores are also easily widened when the bodily fluid is absorbed. Therefore, a larger amount of bodily fluid can be more rapidly taken into the continuous pores, and the composite absorbent body can more reliably exhibit further excellent absorption performance as an absorbent body.

Still another aspect (Aspect 15) of the present invention is:
a sanitary product comprising:
the composite absorbent body according to any of Aspects 1 to 14.

Since the sanitary product of the present aspect includes the composite absorbent body of any one of Aspects 1 to 14, excellent absorption performance can be exhibited as a sanitary product.

### [ADVANTAGEOUS EFFECT OF THE INVENTION]

According to the present invention, a composite absorbent body capable of suppressing a decrease in water absorption amount relative to a pore volume and having excellent absorption performance, and a sanitary product having the same can be provided.

### BRIEF DESCRIPTION OF DRAWINGS

FIG. 1 is a schematic plan view of a light incontinence pad 1 in an unfolded state when viewed in the thickness direction from the skin facing surface side.
FIG. 2 is a diagram illustrating a manufacturing process of an absorbent A which is an example of a polymer absorbent.
FIG. 3 is an SEM photograph of the absorbent A at a magnification of 50 times.
FIG. 4 is an SEM photograph of the absorbent A at a magnification of 100 times. FIG. 5 is an SEM photograph of the absorbent A at a magnification of 500 times.
FIG. 6 is an SEM photograph of the absorbent A at a magnification of 1000 times. FIG. 7 is an SEM photograph of the absorbent A at a magnification of 1500 times. FIG. 8 is a graph showing the relationship between the pore radius of the pores of the absorbent A and the cumulative pore volume.
FIG. 9 is a graph showing the relationship between the pore radius of the pores of the absorbent A and the differential pore volume.
FIG. 10 is a graph showing the relationship between the bulk density and the absorption performance (DW) of the absorbent A.
FIG. 11 is a schematic view showing a measuring apparatus used in a non-pressure DW method.

### DESCRIPTION OF EMBODIMENTS

Hereinafter, a preferable embodiment of a composite absorbent body of the present invention will be described in detail using a light incontinence pad 1 which is an example of a sanitary product to which the composite absorbent body is applied. It should be noted that in the present specification, unless otherwise specified, an "object (for example, a light incontinence pad, a composite absorbent body, or the like) placed on a horizontal plane in an unfolded state is viewed in a thickness direction of the object from the upper side in the vertical direction (in a case where the object is a sanitary product, the top sheet side)" will be simply referred to as a "plan view".

It should be noted that in the present specification, the "lengthwise direction" refers to a "direction in which the length of a longitudinally elongated object (for example, a light incontinence pad, a composite absorbent body, or the like in an unfolded state) in a plan view is long", the "width direction" refers to a "direction in which the length of a longitudinally elongated object in a plan view is short", the "thickness direction" refers to a "direction vertical to the object placed on a horizontal plane in an unfolded state", and the lengthwise direction, the width direction, and the thickness direction have a relationship in which the respective directions are orthogonal to each other.

Further, in the present specification, unless otherwise specified, in the thickness direction of the light incontinence pad 1, a "relatively proximal side with respect to the skin surface of the wearer while the light incontinence pad 1 is put on" will be referred to as a "skin facing surface side," and a "relatively distal side with respect to the skin surface of the wearer while the light incontinence pad 1 is put on" will be referred to as a "non-skin facing surface side."

### [Light Incontinence Pad]

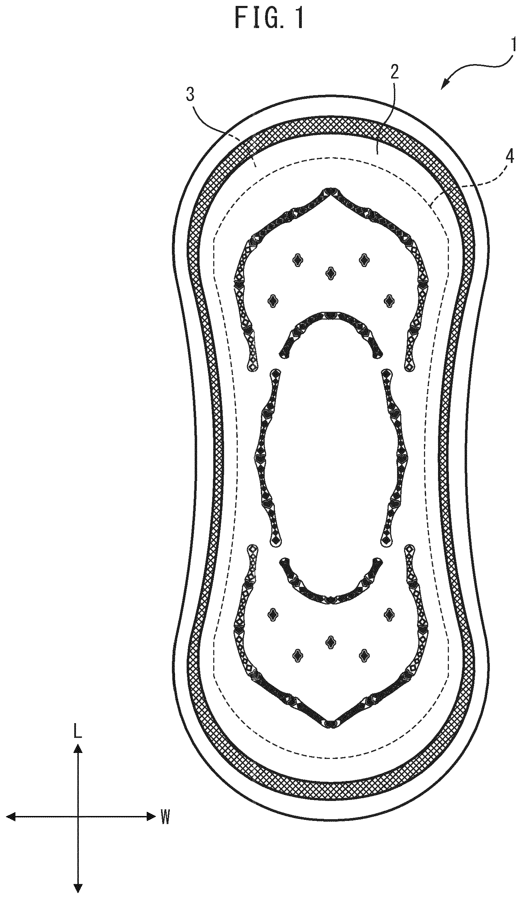

FIG. 1 is a schematic plan view of a light incontinence pad 1 in an unfolded state to which a composite absorbent body 4 according to an embodiment of the present invention is applied. As shown in FIG. 1, in a plan view, the light incontinence pad 1 has a lengthwise direction L and a width direction W, and has a longitudinally elongated outer shape in which two lengthwise end edges protrude toward the outer side in the lengthwise direction in an arc shape. It should be noted that the outer shape of the light incontinence pad 1 is not limited to such an embodiment, and, as long as the light incontinence pad 1 has a longitudinally elongated shape, any shape (for example, an elliptical shape, a rectangular shape, a hourglass shape, a gourd shape, and the like) can be employed according to various uses, usage modes, and the like.

The light incontinence pad 1 basically includes, in a thickness direction, a liquid-permeable top sheet 2 that forms a surface on a skin facing surface side of the light incontinence pad 1, a back sheet 3 that forms a surface on a non-skin facing surface side of the light incontinence pad 1, and a composite absorbent body 4 that is positioned between the top sheet and the back sheet. Further, the light incontinence pad 1 further includes an adhesive portion (not shown) that is arranged on the surface of the non-skin facing surface side of the back sheet 3 and adhesively fixes the light incontinence pad 1 to the inner surface of clothing such underwear of a wearer.

It should be noted that the configuration of the light incontinence pad 1 is not limited to the above-described configuration, and, for example, the light incontinence pad 1 may include a pair of side sheets for forming leakproof walls, which are positioned at both end portions of the light incontinence pad 1 in the width direction W and arranged to extend in the lengthwise direction L, at a position on the skin facing surface side with respect to the top sheet 2, and a plurality of elastic members which are arranged along the lengthwise direction L in each of the pair of side sheets.

Further, in the light incontinence pad 1, the composite absorbent body 4 is formed of a water-absorbent member that is positioned between the top sheet 2 and the back sheet 3 and is capable of absorbing a bodily fluid such as urine or menstrual blood discharged from the wearer and transmitted through the top sheet 2, and the composite absorbent body 4 includes a polymer absorbent having a hydrophilic continuous skeleton and continuous pores, and further includes a superabsorbent polymer (SAP) in the present embodiment. Further, the polymer absorbent exhibits a unique water absorption behavior that, when water is absorbed, the water is taken into the continuous skeleton and then taken into the continuous pores.

In the composite absorbent body 4, when a polymer absorbent having a hydrophilic continuous skeleton and continuous pores described above absorbs a bodily fluid such as urine or menstrual blood, the hydrophilic continuous skeleton instantaneously takes in the bodily fluid by osmotic pressure and expands. Due to this, the volume of the continuous pores can be enlarged, and further the bodily fluid can be taken into the expanded continuous pores. Therefore, a large amount of bodily fluid can be instantaneously absorbed. In the present embodiment, the absorbed bodily fluid can be further transferred to the SAP having a high water retention capacity, and the absorbed bodily fluid can be steadily held in the SAP. Accordingly, the composite absorbent body 4 can exhibit high absorption performance as an absorbent body.

Therefore, the light incontinence pad 1 including such a composite absorbent body 4 can also exhibit excellent absorption performance as a light incontinence pad.

Hereinafter, various configurating members of a sanitary product to which the composite absorbent body of the present invention is applied will be described in more detail using the above-described light incontinence pad 1.

(Top Sheet) In the above-described light incontinence pad 1, the top sheet 2 has a longitudinally elongated outer shape that extends from one side end edge to the other side end edge in the lengthwise direction L of the light incontinence pad 1, and extends over the vicinity of the one side end edge to the vicinity of the other side end edge in the width direction W of the light incontinence pad 1 in a plan view as shown in FIG. 1. The top sheet 2 is formed of a liquid-permeable sheet-like member arranged at a position on the skin facing surface side in the thickness direction of the light incontinence pad 1 and forms a contact surface capable of coming into contact with the skin of the wearer, that is, the surface on the skin facing surface side of the light incontinence pad 1.

Further, as shown in FIG. 1, the top sheet 2 has a slightly larger size in the lengthwise direction L and the width direction W compared with the composite absorbent body 4 arranged on the non-skin facing surface side of the top sheet 2, and is joined to the back sheet 3 positioned on the non-skin facing surface side in the peripheral edge portion.

In the present invention, the outer shape, various dimensions, basis weight, and the like of the top sheet are not particularly limited, as long as they can be used for the top sheet of a sanitary product, and any shape, various dimensions, basis weight, and the like according to the desired liquid permeability, texture, flexibility, strength, and the like can be employed.

(Back Sheet) In the above-described light incontinence pad 1, the back sheet 3 has a longitudinally elongated outer shape that extends from one side end edge to the other side end edge in the lengthwise direction L of the light incontinence pad 1 and extends from the one side end edge to the other side end edge in the width direction W of the light incontinence pad 1 in a plan view. The back sheet 3 is arranged at a position on the non-skin facing surface side in the thickness direction of the light incontinence pad 1, and is formed of a liquid-impermeable sheet-like member that forms the non-skin facing surface of the light incontinence pad 1 and prevents a bodily fluid such as urine or menstrual blood that has permeated the composite absorbent body 4 from leaking out from the light incontinence pad 1.

In the present invention, the outer shape, various dimensions, basis weight, and the like of the back sheet are not particularly limited, as long as they can be used for the back sheet of the sanitary product, and any shape, various dimensions, basis weight, and the like according to the desired leakage preventing performance, breathability, strength, and the like can be employed.

(Composite Absorbent Body) In the above-described light incontinence pad 1, the composite absorbent body 4 has a longitudinally elongated outer shape that extends in a wide region in the lengthwise direction L from the vicinity of one side end edge to the vicinity of the other side end edge in the lengthwise direction L, centering on the central part of the light incontinence pad 1 in the lengthwise direction L and the width direction W, and also extends in the width direction W in a wide region from the vicinity of the one side end edge to the vicinity of the other side end edge in the width direction W , and in which two lengthwise end edges protrude in an arc shape toward the lengthwise outer side in a plan view, as shown in FIG. 1.

More specifically, the composite absorbent body 4 has a constricted portion having a relatively short widthwise length compared with other portions in the lengthwise central part in a plan view, and further has a minimum width portion having the minimum width of the composite absorbent body 4 in the constricted portion, and a maximum width portion having the maximum width of the composite absorbent body 4 on the lengthwise outer side of the constricted portion.

The composite absorbent body 4 is formed of a predetermined water-absorbent member that is arranged between the top sheet 2 and the back sheet 3 in the thickness direction of the light incontinence pad 1 and is capable of absorbing and holding a bodily fluid such as urine or menstrual blood that has permeated the top sheet 2 and the absorbent member is formed of a water-absorbent material such as a polymer absorbent, hydrophilic fibers, or superabsorbent polymer, which will be described later, and a sheet such as a tissue that holds the water-absorbent material. That is, the composite absorbent body means a water-absorbent member formed of a water-absorbent material that can absorb and hold a bodily fluid, and a sheet that holds the water-absorbent material.

It should be noted that in the light incontinence pad 1, the composite absorbent body 4 is joined to each of the top sheet 2 and the back sheet 3 with any adhesive such as a hot-melt adhesive.

Further, the composite absorbent body 4 includes the above-described polymer absorbent having a hydrophilic continuous skeleton and continuous pores and having a unique water absorption behavior, and further includes a superabsorbent polymer in the present embodiment. The polymer absorbent will be described later, but the superabsorbent polymer is a powder or granular matter formed of a superabsorbent polymer such as a sodium acrylate copolymer well-known in the art, and is referred to as super absorbent polymer (SAP).

It should be noted that the composite absorbent body 4 may include only the above-described polymer absorbent, or may include only the polymer absorbent and the superabsorbent polymer as the water-absorbent material, or may further include a water-absorbent material well-known in the art in addition to these materials. As such a water-absorbent material, there are provided hydrophilic fibers and the like, and more specifically, cellulose-based fibers such as pulp fibers (for example, pulverized pulp), cotton, rayon, and acetate and the like can be used.

It should be noted that the composite absorbent body 4 may have a configuration in which such a polymer absorbent, a superabsorbent polymer, and any water-absorbent material are covered with a wrap sheet such as a tissue having hydrophilicity.

It should be noted that, in the present invention, the outer shape, various dimensions, basis weight, and the like of the composite absorbent body are not particularly limited as long as the effects of the present invention are not impaired, and any outer shape, various dimensions, basis weight, and the like according to the desired water absorption, flexibility, strength, and the like can be adopted.

Hereinafter, the polymer absorbent used in the composite absorbent body of the present invention will be described in more detail.

### [Polymer Absorbent]

In the present invention, the polymer absorbent is not particularly limited as long as the absorbent has a hydrophilic continuous skeleton and continuous pores, and exhibits a unique water absorption behavior that, when water is absorbed, the water is taken into the continuous skeleton and then taken into the continuous pores. As such a polymer absorbent, for example, there is provided a polymer compound that is a hydrolysate of a crosslinked polymer of two or more monomers containing at least a (meth)acrylic acid ester and has at least one or more hydrophilic groups in the functional group. More specifically, there is provided a polymer compound that is a hydrolysate of a crosslinked polymer of a (meth)acrylic acid ester and a compound containing two or more vinyl groups in one molecule and has at least -COONa group. Such a polymer absorbent is an organic porous body having at least one or more -COONa groups in one molecule and may further have a -COOH group. In the skeleton of the porous body, -COONa groups are substantially uniformly distributed.

When the polymer absorbent is a polymer compound that is a hydrolysate of a crosslinked polymer of such (meth)acrylic acid ester and a compound containing two or more vinyl groups in one molecule and contains at least one or more a -COONa groups, as described later, the hydrophilic continuous skeleton easily extends (that is, easily expands) when a bodily fluid such as urine or menstrual blood is absorbed, and the continuous pores are easily widened. Therefore, a larger amount of bodily fluid can be more rapidly taken into the continuous pores, and further excellent absorption performance can be exhibited as an absorbent body.

It should be noted that in the present specification, the term "(meth)acrylic acid ester" refers to an acrylic acid ester or a methacrylic acid ester.

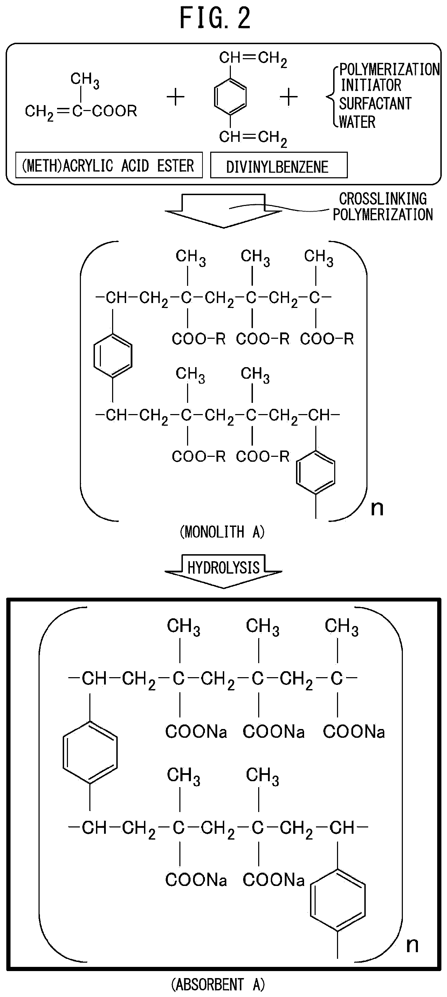

In the polymer absorbent formed of such a hydrolysate of a crosslinked polymer of a (meth)acrylic acid ester and divinylbenzene, a hydrophilic continuous skeleton is formed of an organic polymer having at least a -COONa group, and has continuous holes (continuous pores) that serve as absorption sites for a liquid to be absorbed (that is, a bodily fluid such as urine or menstrual blood) are provided between the skeletons. It should be noted that since in the hydrolysis treatment, the - COOR group (that is, the carboxylic acid ester group) of the crosslinked polymer is converted into a -COONa group or a -COOH group (refer to FIG. 2), the polymer absorbent may have a -COOR group.

The presence of the -COOH group and the -COONa group in the organic polymer that forms the hydrophilic continuous skeleton can be confirmed by analysis through infrared spectrophotometry or a method of quantifying a weakly acidic ion exchange group.

Here, FIG. 2 is a diagram illustrating a manufacturing process of an absorbent A which is an example of a polymer absorbent. In FIG. 2, the upper figure shows polymer constituent raw materials, the middle figure shows a monolith A as a crosslinked polymer of a (meth)acrylic acid ester and divinylbenzene, and the lower figure shows the absorbent A obtained by subjecting the monolith A in the middle figure to hydrolysis and drying.

Hereinafter, an absorbent A formed of a hydrolysate of a crosslinked polymer of a (meth)acrylic acid ester and divinylbenzene, which is an example of a polymer absorbent, will be described.

The polymer absorbent is a hydrolysate of a crosslinked polymer of a (meth)acrylic acid ester and a compound having two or more vinyl groups in one molecule, or a hydrolysate of a crosslinked polymer of two or more types of monomers containing at least a (meth)acrylic acid ester, or the like. However, when the polymer absorbent is a monolithic absorbent, there are advantages that the bodily fluid can be rapidly absorbed and the bodily fluid temporarily held in the polymer absorbent can be more steadily transferred to the SAP.

It should be noted that in the following description, the "monolith A" is an organic porous body formed of a crosslinked polymer of a (meth)acrylic acid ester and divinylbenzene before being subjected to hydrolysis, and will also be referred to as a "monolithic organic porous body". The "absorbent A" is a hydrolysate of a crosslinked polymer (monolith A) of a (meth)acrylic acid ester and divinylbenzene after being subjected to hydrolysis and drying. It should be noted that the absorbent A is assumed to be in the dry state in the following description.

First, the structure of the absorbent A will be described. As described above, the absorbent A has a hydrophilic continuous skeleton and continuous pores. The absorbent A, which is an organic polymer having a hydrophilic continuous skeleton, is obtained by performing crosslinking polymerization using a (meth)acrylic acid ester, which is a polymerization monomer, and divinylbenzene, which is a crosslinking monomer, and then hydrolyzing the obtained crosslinked polymer (monolith A) as shown in FIG. 2.

The organic polymer that forms the hydrophilic continuous skeleton has, as constituent units, polymerization residue of an ethylene group (hereinafter, referred to as a "constituent unit X") and a crosslinked polymerization residue of divinylbenzene (hereinafter, referred to as a "constituent unit Y"). Further, the polymerization residue (constituent unit X) of the ethylene group in the organic polymer that forms the hydrophilic continuous skeleton has a -COONa group or both of the -COOH group and the -COONa group generated by the hydrolysis of the carboxylic acid ester group. It should be noted that, in a case where the polymerization monomer is a (meth)acrylic acid ester, the polymerization residue of the ethylene group (constituent unit X) has a -COONa group, a -COOH group, and an ester group.

In the absorbent A, the ratio of the crosslinked polymerization residue (constituent unit Y) of divinylbenzene in the organic polymer that forms the hydrophilic continuous skeleton is, for example, 0.1 to 30 mol%, and preferably 0.1 to 20 mol%, with respect to all of the constituent units. For example, in the absorbent A containing butyl methacrylate as the polymerization monomer and divinylbenzene as the crosslinking monomer, the ratio of the crosslinked polymerization residue (constituent unit Y) of divinylbenzene in the organic polymer that forms the hydrophilic continuous skeleton is, for example, about 3 mol%, preferably 0.1 to 10 mol%, and more preferably 0.3 to 8 mol%, with respect to all constituent units. It should be noted that, when the ratio of the crosslinked polymerization residue of divinylbenzene in the organic polymer that forms the hydrophilic continuous skeleton is 0.1 mol% or more, it becomes difficult to decrease the strength of the absorbent A, and, when the ratio of the crosslinked polymerization residue of divinylbenzene is 30 mol% or less, it becomes difficult to decrease the absorption amount of the liquid to be absorbed.

In the absorbent A, the organic polymer that forms the hydrophilic continuous skeleton may be formed of only the constituent unit X and the constituent unit Y, or may be formed of, in addition to the constituent unit X and the constituent unit Y, a constituent unit other than the constituent unit X and the constituent unit Y, such as a polymerization residue of monomers other than (meth)acrylic acid ester and divinylbenzene.

As the constituent unit other than the constituent unit X and the constituent unit Y, for example, there are provided polymerization residues of monomers such as styrene, α-methylstyrene, vinyltoluene, vinylbenzyl chloride, glycidyl (meth)acrylate, isobutene, butadiene, isoprene, chloroprene, vinyl chloride, vinyl bromide, vinylidene chloride, tetrafluoroethylene, (meth)acrylonitrile, vinyl acetate, ethylene glycol di(meth)acrylate, tripropylene glycol di(meth)acrylate, trimethylol propane tri(meth)acrylate and the like.

It should be noted that the ratio of the constituent unit other than the constituent unit X and the constituent unit Y in the organic polymer that forms the hydrophilic continuous skeleton is, for example, 0 to 50 mol%, and preferably 0 to 30 mol%, with respect to all of the constituent units.

Further, it is preferable that the thickness of the hydrophilic continuous skeleton of the absorbent A is 0.1 to 100 µm. When the thickness of the hydrophilic continuous skeleton of the absorbent A is 0.1 µm or more, spaces (pores) for taking in a liquid (bodily fluid) to be absorbed in the porous body is less likely to collapse during absorption, and the absorption amount is less likely to decrease. On the other hand, when the thickness of the hydrophilic continuous skeleton is 100 µm or less, an excellent absorption rate can be easily obtained. However, the thickness of the continuous skeleton can be obtained from, for example, an SEM observation photograph of the absorbent A.

It should be noted that since the pore structure of the hydrophilic continuous skeleton of the absorbent A is an open cell structure, the thickness of the continuous skeleton is measured at the thickness evaluation point, which is the cross section of the skeleton appearing in a test piece for electron microscope measurement. The continuous skeleton is formed by spaces between water droplets removed by dehydration and drying after hydrolysis described later, and therefore the skeleton is often polygonal. Therefore, the thickness of the continuous skeleton is defined as the average value of the diameter (µm) of a circle that circumscribes the polygonal cross section. In addition, in rare cases, a small hole may exist in the polygon, but in such cases, the circumscribed circle of the polygonal cross section that surrounds the small hole is measured.

Further, it is preferable that the average diameter of the continuous pores of the absorbent A is 1 to 1000 µm. When the average diameter of the continuous pores of the absorbent A is 1 µm or more, the spaces (pores) for taking in the liquid (bodily fluid) to be absorbed in the porous body become less likely to collapse during absorption, and the absorption rate is less likely to decrease. On the other hand, when the average diameter of the continuous pores is 1000 µm or less, an excellent absorption rate can be easily obtained.

It should be noted that the average diameter (µm) of the continuous pores of the absorbent A can be measured by mercury intrusion porosimetry, and the maximum value of the pore distribution curve obtained by such a mercury intrusion porosimetry is employed. For a sample used for measurement of the average diameter of the continuous pores, regardless of the ionic form of the absorbent A, a sample dried in a vacuum dryer set at a temperature of 50°C for 18 hours or longer is used. It should be noted that the final reaching pressure is set to 0 Torr.

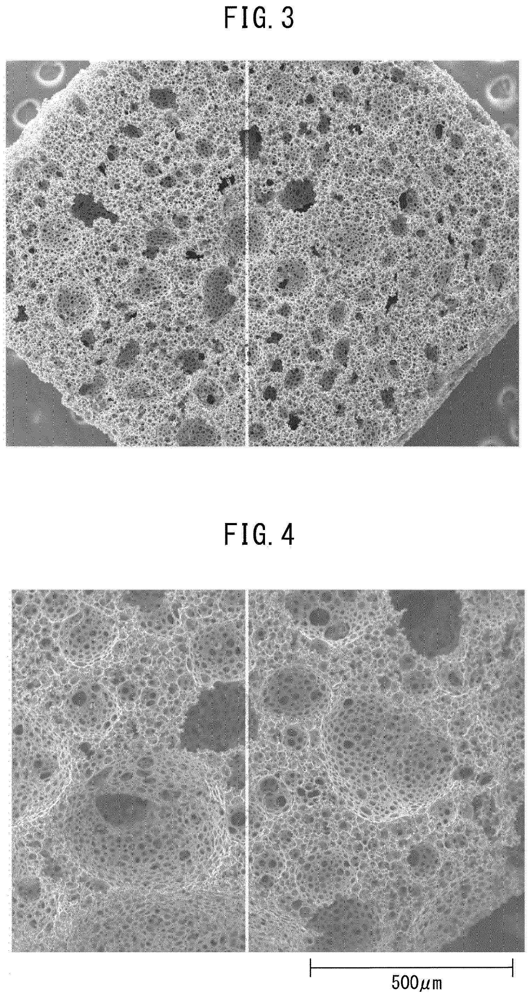

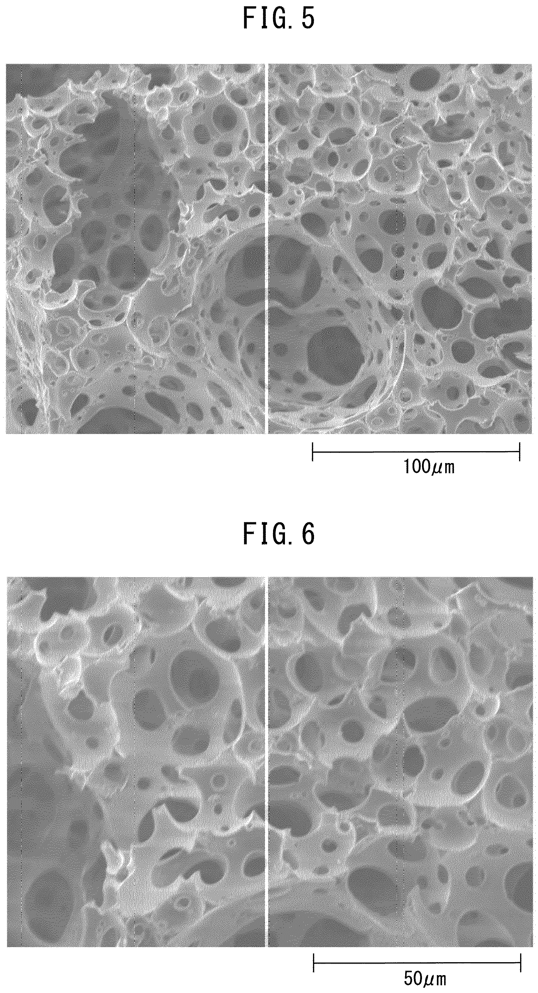

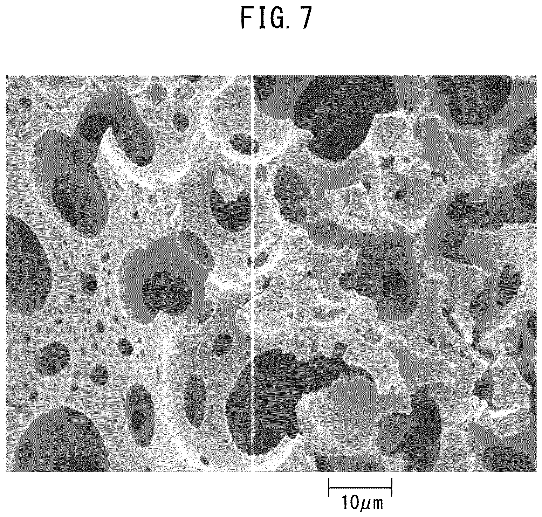

Here, FIG. 3 is an SEM photograph of the absorbent A at a magnification of 50 times, FIG. 4 is an SEM photograph of the absorbent A at a magnification of 100 times, FIG. 5 is an SEM photograph of the absorbent A at a magnification of 500 times, FIG. 6 is an SEM photograph of the absorbent A at a magnification of 1000 times, and FIG. 7 is an SEM photograph of the absorbent A at a magnification of 1500 times. The absorbent A shown in FIGS. 3 to 7 is an example of an absorbent containing butyl methacrylate as the polymerization monomer and divinylbenzene as the crosslinking monomer, and has a cubic structure having 2 mm sides.

The absorbent A shown in FIGS. 3 to 7 has a large number of bubble-shaped macropores, and further has a portion in which these bubble-shaped macropores overlap each other. The absorbent A has an open cell structure in which portion in which these macropores overlap each other forms a common opening (mesopore), that is, an open cell structure (continuous macropore structure).

The portion in which the macropores overlap each other forms a common opening (mesopore) having an average diameter of 1 to 1000 µm in a dry state, preferably 10 to 200 µm, and particularly preferably 20 to 100 µm, and most of the common openings (mesopores) have an open pore structure. When the average diameter of the mesopores in the dry state is 1 µm or more, the absorption rate of the liquid to be absorbed becomes more favorable. On the other hand, when the average diameter of the mesopores in the dry state is 1000 µm or less, the absorbent A is less likely to be embrittled. It should be noted that such an overlap between the macropores is approximately 1 to 12 pieces in one macropore, and is approximately 3 to 10 pieces in many cases.

Further, the absorbent A having such an open cell structure makes it possible to uniformly form a macropore group and a mesopore group, and also has the advantage of making it possible to remarkably increase the pore volume and specific surface area compared with a particle agglomerated porous body such as that described in Japanese Patent Application Publication No. H08-252579.

The continuous pores included in the absorbent A are a plurality of pores that are continuous with each other. The total pore volume of the pores of the absorbent A is preferably 0.5 to 50 mL/g, more preferably 0.9 to 40 mL/g, and still more preferably 2 to 30 mL/g. When the total pore volume of the absorbent A is 0.5 mL/g or more, a sufficient pore volume can be secured in the absorbent A, and therefore a sufficient water absorption amount can be secured. Further, the spaces (pores) for taking in the liquid to be absorbed (bodily fluid) in the porous body can be made less likely to collapse during absorption, and the water absorption amount and the water absorption rate can be made less likely to decrease. On the other hand, when the total pore volume of the absorbent A is 50 mL/g or less, the strength of the absorbent A can be made less likely to decrease.

It should be noted that the total pore volume can be measured by the mercury intrusion porosimetry. As a sample for measurement of the total pore volume, regardless of the ionic form of the absorbent A, a sample obtained by drying in a vacuum dryer set at a temperature of 50°C for 18 hours or longer is used. The final ultimate pressure is assumed to be 0 Torr. The cumulative (integrated) pore volume distribution (the relationship between the pore radius and the cumulative pore volume), the log differential pore volume distribution (the relationship between the pore radius and the log differential pore volume), and the like are obtained by the mercury intrusion method, and the total pore volume (mL/g), the average pore radius (µm), the maximum pore radius (µm), and the pore volume (mL/g) and the ratio (%) of pores having a predetermined pore radius or more (/or less), the pore volume variation coefficient, and the like can be calculated. It should be noted that the maximum pore diameter (µm) refers to the pore radius of the pore showing the maximum value of the pore volume. In addition, as for the pore volume (mL/g) at each pore radius, the log differential pore volume (mL/g) in the log differential pore volume distribution is adopted.

Here, in the pores of the absorbent A, the ratio of the pore volume of the pores having a pore radius of 1 µm or more is 90% or more of the pore volume (total pore volume) of all of the pores, preferably 93% or more, and more preferably 95% or more. When the ratio of the pore volume of the pores having a pore radius of 1 µm or more is 90% or more, during water absorption, water is less likely to enter the pores having a relatively small pore radius such as a pore radius of less than 1 µm, and even when water cannot enter the pores, a sufficient water absorption amount can be secured. Accordingly, a decrease in the water absorption amount relative to the pore volume can be suppressed, and excellent absorption performance can be obtained.

In the pores of the absorbent A, the ratio of the pore volume of the pores having a pore radius of 0.005 µm or less is preferably less than 10% of the pore volume (total pore volume) of all of the pores, and the ratio of the pore volume of the pores having a pore radius of 0.05 µm or less is more preferably less than 10% of the pore volume (total pore volume) of all of the pores. In the absorbent A, the ratio of the pore volume of the pores having a very small pore radius, such as a pore radius of 0.005 µm or less and difficult to absorb water is very small, and the ratio of the pore volume of pores having a large pore radius, such as a pore radius of 1 µm or more, and capable of absorbing water is large (90% or more). Accordingly, the pores of the absorbent A can be effectively utilized for water absorption, and a sufficient water absorption amount can be secured.

In addition, in the pores of the absorbent A, the pore radius in the pore showing the maximum value of the pore volume is preferably 0.5 µm or more and 500 µm or less, more preferably 300 µm or less, and still more preferably 150 µm or less. When the pore radius at the maximum value of the pore volume is 500 µm or less, during water absorption, the breakage (collapse) of the structure of the continuous skeleton of the absorbent A can be suppressed, an excellent absorption rate is easily obtained, and a sufficient water absorption amount can be stably secured. In a case where the pore radius is 500 µm or more at the maximum value of the pore volume, there is a risk that the structure of the continuous skeleton cannot be maintained and collapses during water absorption.

In addition, in the pores of the absorbent A, the pore distribution (pore volume) variation coefficient in the pores having a pore radius of 1 µm or more may be 1.4 or less. In a case where the variation coefficient of the pore distribution is 1.4 or less, the variation of the pore radius with respect to the average value of the pore radii is small, and the peak indicated by the pore distribution becomes sharp in the vicinity of the average value of the pore radii. Therefore, the absorbent A can absorb water substantially uniformly from the entire surface and in all directions. Accordingly, the pores of the polymer absorbent can be effectively utilized for water absorption, and a sufficient water absorption amount can be secured.

On the other hand, in the pores of the absorbent A, the pore distribution (pore volume) variation coefficient in the pores having a pore radius of 1 µm or more may be more than 1.4. In this case, since the variation coefficient of the pore distribution is more than 1.4, the variation of the pore radius with respect to the average value of the pore radii is large, and the peak indicated by the pore distribution becomes broad in the vicinity of the average value of the pore radii. That is, the absorbent A has pores having a small pore radius and pores having a large pore radius. Therefore, in the pores having a small pore radius, a capillary force easily acts, and thus the water absorption rate is easily increased. In the pores having a large pore radius, the water absorption volume is easily increased. Therefore, due to two synergistic effects, the absorbent A can instantly absorb a large amount of water inside the pores.

Here, in the pores of the absorbent A, in a case where the peak indicated by the pore distribution is broad, a portion on the side with a larger pore radius may be broader than a portion on the side with a smaller pore radius with respect to the pore radius corresponding to the local maximum value of the pore volume in the curved line showing the pore distribution. In this case, the absorbent A has a configuration in which there are more pores having a larger pore diameter than pores having a smaller pore radius. Accordingly, since there are a large number of pores having a large pore radius, the water absorption volume is more easily increased, and a larger amount of water can be absorbed inside the pores.

On the other hand, in the pores of the absorbent A, even in a case where the peak indicated by the pore distribution is broad, a portion on the side with a smaller pore radius may be broader than a portion on the side with a larger pore radius with respect to the pore radius corresponding to the local maximum value of the pore volume in the curved line showing the pore distribution. In this case, the absorbent A has a configuration in which there are more pores having a smaller pore diameter than pores having a larger pore radius. The large number of pores having a small pore radius makes the capillary force more likely to work, the water absorption rate is more easily increased, and water can be more instantaneously absorbed inside the pores.

Further, in the pores of the absorbent A, at least two local maximum values of the pore volume may be present in the curved line showing the pore distribution. In this case, in the absorbent A, there are pores having a predetermined small pore radius and a pore radius in the vicinity thereof, and pores having a predetermined large pore radius and a pore radius in the vicinity thereof. Therefore, in the pores having a relatively small pore radius, a capillary force easily acts, and thus the water absorption rate is easily increased. In the pores having a relatively large pore radius, the water absorption volume is easily increased. Therefore, due to two synergistic effects, a polymer absorbent can instantaneously absorb a large amount of water inside the pores.

Here, in the pores of the absorbent A, in a case where there are two local maximum values of the pore volume, of the two local maximum values of the pore volume in the curved line showing the pore distribution, a relatively small local maximum value of the pore radius may be larger than a relatively large local maximum value of the pore radius. In this case, in the absorbent A, there are more pores having a smaller pore diameter than pores having a larger pore radius. The large number of pores having a small pore radius makes the capillary force more likely to work, the water absorption rate is more easily increased, and water can be more instantaneously absorbed inside the pores.

On the other hand, in the pores of the absorbent A, even in a case where there are two local maximum values of the pore volume, of the two local maximum values of the pore volume in the curved line showing the pore distribution, a relatively small local maximum value of the pore volume may be smaller than a relatively large local maximum value of the pore volume. In this case, in the absorbent A, there are more pores having a larger pore diameter than pores having a smaller pore radius. Since there are a large number of pores having a large pore radius, the water absorption volume is more easily increased, and a larger amount of water can be absorbed inside the pores.

In addition, in the pores of the absorbent A, the bulk density is preferably 0.07 to 0.6 g/cm³, more preferably 0.1 to 0.4 g/cm³, and still more preferably 0.15 to 0.35 g/cm³. In that case, as will be described later, the water absorption rate (DW) performance can be set to 6 mL/30 sec. or more, and is more preferably 10 mL/30 sec. or more, and still more preferably 12 mL/30 sec. or more. That is, since the water absorption rate is high, the polymer absorbent can more instantaneously absorb water inside the pores.

### <Method for Measuring Water Absorption Rate (DW)>

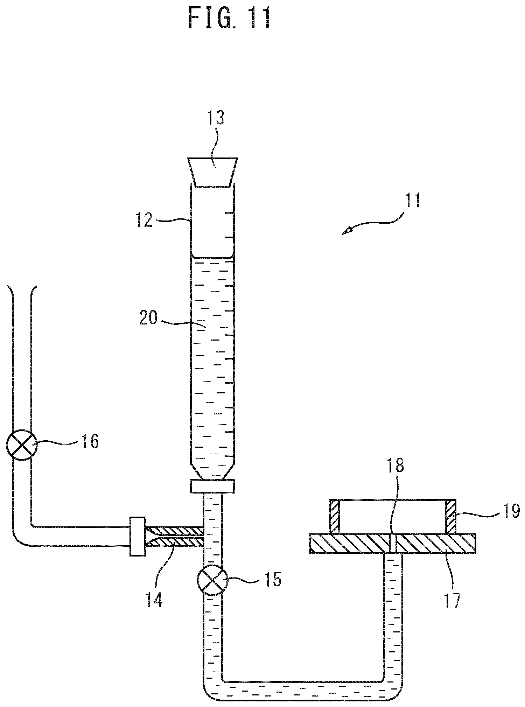

The water absorption rate of the absorbent is measured by a non-pressure DW (Demand Wettability) method. FIG. 11 is a schematic view showing a measuring apparatus used in the non-pressure DW method. As such a measuring apparatus, a DW apparatus 11 (Demand Wettability apparatus, manufactured by Taiyo Create Co., Ltd.) is used. As shown in the drawing, the DW apparatus 11 includes a burette 12 (a scale volume of 50 ml, a length of 86 cm, an inner diameter of 1.05 cm), a rubber stopper 13, an air inlet tube (leading end inner diameter of 3 mm) 14, a cock 15, a cock 16, a measuring table 17, a liquid outlet (inner diameter of 3 mm) 18, a cylinder 19, and a test solution 20. A conduit (inner diameter: 7 mm) is attached to the burette 12 to the measuring table 17. The test solution used is a 0.9% sodium chloride aqueous solution. The measurement is performed in an atmosphere of 25°C × 50% humidity RH (in a constant temperature and humidity room).

The measurement procedure is as follows.
(1) The test solution 20 is added to point 0 or higher (the top (0 ml line) of the burette 12 scale), with both the cocks 15 and 16 in the DW apparatus 11 closed, and the rubber stopper 13 is placed on the upper part of the burette 12 to seal the burette.
(2) After placing filter paper on the liquid outlet 18 of the measuring table 17, both the cocks 15 and 16 are opened, and the liquid level is adjusted to point 0 while suctioning out the liquid flowing out from the liquid outlet 18 with the filter paper. After adjusting, the cocks 15 and 16 are closed.
(3) A tissue made of 100% wood pulp (basis weight: 15 ± 1 gsm, thickness when measured with a nonwoven fabric thickness meter at a measurement pressure of 3 g/cm²: 0.1 ± 0.02 mm) is placed on the measuring table 17 in a manner such that the liquid outlet 18 is located at the center.
(4) A cylinder 19 having a diameter of 30 mm is at the central part of the tissue, and an object to be tested (polymer absorbent) is placed in the cylinder 19 with the liquid outlet 18 as the center. The object to be tested is placed in the cylinder 19, the circumference of which is constrained by the cylinder 19.
(5) The measurement start time is defined as the time when the cocks 15 and 16 are opened, the object to be tested starts to absorb the test solution 20, and the first bubble introduced from the air inlet tube 14 reaches the water surface of the test solution 20 in the burette 12 (at the time when the water surface of the test solution 20 in the burette 12 is lowered).
(6) Continuously, the reduced amount of M (ml) of the test solution 20 in the burette 12 (the amount of the test solution 20 absorbed by the object to be tested) is read.
(7) The amount of the object to be tested absorbed after a predetermined time has elapsed from the start of liquid absorption (in the present embodiment, after 30 seconds have elapsed) is determined by the DW method: the amount (ml/g) of the object to be tested absorbed = M (ml)/(the weight (g) of the object to be tested (polymer absorbent)).

Hereinafter, the appearance in a case where the absorbent A and a liquid such as a bodily fluid (hereinafter, simply referred to as a "bodily fluid") come into contact with each other will be described. However, the same applies to a case where the composite absorbent body 4 containing the absorbent A comes into contact with the bodily fluid. Further, since the mass of the absorbed bodily fluid is substantially proportional to the volume of the bodily fluid, in the following description, the mass of the bodily fluid is sometimes simply referred to as the "amount of bodily fluid".

As shown in FIGS. 3 to 7, the continuous pores of the absorbent A are pores in which a plurality of pores are continuous with each other, and the fact that a large number of pores are located can be visually recognized from the external appearance as well. When the bodily fluid such as urine or menstrual blood comes into contact with the absorbent A having a large number of pores, the hydrophilic continuous skeleton first instantaneously takes in a part of the bodily fluid by osmotic pressure and extends (that is, expands). The continuous skeleton extends in substantially all directions. The absorbent A which is increased due to the absorption of a predetermined amount of bodily fluid in this manner is capable of further absorbing a predetermined amount of bodily fluid into the expanded continuous pores due to the capillary phenomenon. The absorbent A exhibits a unique water absorption behavior that, when water (bodily fluid) is absorbed, the water is taken into the hydrophilic continuous skeleton and then taken into the continuous pores to be absorbed.

It should be noted that, while the bodily fluid absorbed into the hydrophilic continuous skeleton of the absorbent A is less likely to be released from the continuous skeleton (that is, less likely to be separated), the bodily fluid absorbed into the continuous pores is likely to be separated. Therefore, in the composite absorbent body, the bodily fluid absorbed into the continuous pores is separated, transferred to a superabsorbent polymer (SAP) having a high water retention capacity, and steadily held in the SAP. Here, regarding the amount of bodily fluid absorbed into the continuous skeleton of the absorbent A and the amount of bodily fluid absorbed into the continuous pores, in the total amount of bodily fluid absorbed by the absorbent A, the amount of bodily fluid separated from the absorbent A (the amount of separation) in a centrifugation treatment (at 150 G for 90 seconds) becomes the amount of bodily fluid absorbed into the continuous pores, and the amount of bodily fluid excluding the amount of bodily fluid (that is, the amount of bodily fluid not separated from the absorbent A in the centrifugation treatment) becomes the amount of bodily fluid absorbed into the continuous skeleton.

Further, in the bodily fluid absorbed by the absorbent A, the amount of bodily fluid that remains in the pores is larger than the amount of bodily fluid absorbed in the hydrophilic continuous skeleton. Since most of the bodily fluid absorbed in the absorbent A is absorbed by storing the bodily fluid in the pores due to the capillary phenomenon, the higher the porosity (the void volume of the pores with respect to the volume of the absorbent A), which is the ratio of the void volume of the pores (total pore volume), the more the bodily fluid can be absorbed. It should be noted that it is preferable that the porosity is 85% or more.

For example, when the porosity of the absorbent A shown in FIGS. 3 to 7 described above is calculated, the following is obtained. First, the specific surface area of the absorbent A obtained by the mercury intrusion porosimetry is 400 m²/g, and the pore volume is 15.5 mL/g. This pore volume of 15.5 mL/g means that the volume of the pores in 1 g of the absorbent A is 15.5 mL. In a case where the specific gravity of the absorbent A is 1 g/mL, the volume of the pores occupied in 1 g of the absorbent A, that is, the pore volume, is 15.5 mL, and the volume of 1 g of the absorbent A is 1 mL. Then, the total volume (volume) of 1 g of the absorbent A becomes 15.5 + 1 (mL), and the ratio of the pore volume in the total volume becomes the porosity. Therefore, the porosity of the absorbent A becomes 15.5/(15.5 + 1) x 100 ≅ 94%.

The absorbent A having such a hydrophilic continuous skeleton and continuous pores, that is, the polymer absorbent is applied to a composite absorbent body for absorbing a bodily fluid such as urine or menstrual blood, such as the composite absorbent body 4 of the light incontinence pad 1 described above, in the form of, for example, a particle, a sheet, or the like. Further, as described above, this polymer absorbent exhibits a unique water absorption behavior that when water is absorbed, the water is taken into the hydrophilic continuous skeleton and then taken into the continuous pores. Therefore, a large amount of bodily fluid can be instantaneously absorbed, and further the absorbed bodily fluid (mainly the bodily fluid absorbed in the continuous pores) can be transferred to the SAP having a high water retention capacity and can be steadily held in the SAP. Therefore, the composite absorbent body to which such a polymer absorbent is applied can exhibit high absorption performance as an absorbent body.

However, the water absorption amount of the polymer absorbent can be measured according to the following method for measuring the water absorption amount of the polymer absorbent.

### <Method for Measuring Water Absorption Amount of Polymer Absorbent>

(1) 1 g of a sample for measurement (polymer absorbent) is sealed in a mesh bag (N-No255HD 115 (standard width: 115 cm, 255 mesh/2.54 cm, opening: 57 µm, wire diameter: 43 µm, thickness: 75 µm), manufactured by NBC Meshtec Inc.) which is cut into 10 cm squares. It should be noted that the mass (g) of the mesh bag is measured in advance. Further, the measurement method is performed under conditions of a temperature of 25°C and a humidity of 60%. Further, in a case where the sample for measurement (polymer absorbent) is collected from a product of a sanitary product and used, the sample for measurement (polymer absorbent) can be obtained according to the above-described <Method for Collecting Sample for Measurement (Polymer Absorbent)>.
(2) The mesh bag containing the sample is immersed in physiological saline (0.9% sodium chloride aqueous solution) for 1 hour.
(3) The mass (g) after the mesh bag is suspended for 5 minutes and drained is measured.
(4) The water absorption amount (g) of the sample is calculated by subtracting the mass (= 1 g) of the sample and the total mass of the mesh bag from the mass of the mesh bag after draining measured in (3) above, and the water absorption amount is further divided by the mass (= 1 g) of the sample to obtain the water absorption amount (g/g) per unit mass of the sample (polymer absorbent).

It should be noted that in a case where the sample for measurement (polymer absorbent) is collected from a product of a sanitary product and used, the sample for measurement (polymer absorbent) can be obtained according to the following <Method for Collecting Sample for Measurement (Polymer Absorbent)>.

### <Method for Collecting Sample for Measurement (Polymer Absorbent)>

(1) The top sheet or the like is peeled off from the product of the sanitary product to expose the absorbent body.
(2) The object to be measured (polymer absorbent) is dropped from the exposed absorbent body, and a substance other than the object to be measured (in a particulate state) (for example, pulp, synthetic resin fibers, or the like) is removed with tweezers or the like.
(3) A microscope or a simple loupe is used as magnifying observation means, and the object to be measured is collected using the tweezers or the like, while observing at a magnification capable of recognizing the difference from SAP or at a magnification capable of visually recognizing the pores of the porous body. It should be noted that the magnification of the simple loupe is not particularly limited as long as the pores of the porous body can be visually recognized, and, for example, a magnification of 25 to 50 times can be given.
(4) The object to be measured collected in this manner is used as the sample for measurement in various measurement methods.

Hereinafter, a method for manufacturing such a polymer absorbent will be described in detail using the above-described absorbent A as an example.

### [Method for Manufacturing Polymer Absorbent]

As shown in FIG. 2, the above-described absorbent A can be obtained by allowing the absorbent A to pass through a crosslinking polymerization step and a hydrolysis step. Hereinafter, each of these steps will be described.

(Crosslinking Polymerization Step) First, an oil-soluble monomer for crosslinking polymerization, a crosslinking monomer, a surfactant, water, and optionally a polymerization initiator are mixed to obtain a water-in-oil emulsion. This water-in-oil emulsion is an emulsion in which water droplets are dispersed in an oil phase that serves as a continuous phase.

As shown in the upper figure of FIG. 2, in the above-described absorbent A, a monolith A is obtained by performing crosslinking polymerization using butyl methacrylate, which is (meth)acrylic acid ester, as an oil-soluble monomer, divinylbenzene as a crosslinking monomer, sorbitan monooleate as a surfactant, and further isobutyronitrile as a polymerization initiator.

Specifically, in the absorbent A, as shown in the upper figure of FIG.2, first, 9.2 g of t-butyl methacrylate as an oil-soluble monomer, 0.28 g of divinylbenzene as a crosslinking monomer, 1.0 g of sorbitan monooleate (hereinafter, abbreviated as "SMO") as a surfactant, and 0.4 g of 2,2'-azobis(isobutyronitrile) as a polymerization initiator are mixed and uniformly dissolved. Next, the mixture of t-butyl methacrylate, divinylbenzene, SMO, and 2,2'-azobis(isobutyronitrile) is added to 180 g of pure water, and a vacuum stirring defoaming mixer (manufactured by EME. Corp.), which is a planetary motion type stirring device, is used to perform stirring under reduced pressure to obtain a water-in-oil emulsion.

Further, the emulsion is rapidly transferred to a reaction vessel, sealed, and left to stand still and polymerized at 60°C for 24 hours. After completion of the polymerization, the content is taken out, extracted with methanol, and dried under reduced pressure to obtain a monolith A having a continuous macropore structure. It should be noted that, as a result of observing the internal structure of the monolith A by SEM, the monolith A had an open cell structure and the thickness of the continuous skeleton is 5.4 µm. Further, the average diameter of the continuous pores measured by the mercury intrusion porosimetry is 36.2 µm, and the total pore volume is 15.5 mL/g.

It should be noted that the content of divinylbenzene with respect to all the monomers is preferably 0.3 to 10 mol%, and more preferably 0.3 to 5 mol%. Further, the ratio of divinylbenzene to the total of butyl methacrylate and divinylbenzene is preferably 0.1 to 10 mol%, and more preferably 0.3 to 8 mol%. It should be noted that in the above-described absorbent A, the ratio of butyl methacrylate with respect to the total of butyl methacrylate and divinylbenzene is 97.0 mol% and the ratio of divinylbenzene with respect to the total of butyl methacrylate and divinylbenzene is 3.0 mol%.

The amount of the surfactant added can be set depending on the type of the oil-soluble monomer and the size of the desired emulsion particle (macropore), and it is preferable that the amount of the surfactant is set to be in a range of about 2 to 70% with respect to the total amount of the oil-soluble monomer and the surfactant.

It should be noted that, in order to control the bubble shape and the size of the monolith A, alcohols such as methanol and stearyl alcohol; carboxylic acids such as stearic acid; hydrocarbons such as octane, dodecane, and toluene; cyclic ethers such as tetrahydrofuran and dioxane, and the like may coexist in the polymerization system.

Further, the mixing method for forming the water-in-oil emulsion is not particularly limited, and for example, any mixing method such as a method of mixing each component all together at once, and a method in which oil-soluble components, which are an oil-soluble monomer, a surfactant, and an oil-soluble polymerization initiator, and water-soluble components, which are water or a water-soluble polymerization initiator, are uniformly dissolved separately, and then oil-soluble components and water-soluble components are mixed can be adopted.

Further, the mixing device for forming the emulsion is not particularly limited, and any device such as an ordinary mixer, homogenizer, or high pressure homogenizer can be used according to the desired emulsion particle diameter. Further, a so-called planetary type stirring device in which an object to be treated is placed in a mixing container, and the object to be treated is stirred and mixed by rotating the mixing container in a tilted state while revolving around the revolution axis can be used.

Further, the mixing conditions are not particularly limited, and any stirring rotation rate, any stirring time, and the like can be set depending on the desired emulsion particle diameter. It should be noted that, in the above-described planetary type stirring device, water droplets can be uniformly generated in the W/O emulsion, and any average diameter can be set within a wide range.

As the polymerization conditions of the water-in-oil emulsion, various conditions can be adopted depending on the type of monomer or initiator and the like. For example, in a case of using azobisisobutyronitrile, benzoyl peroxide, potassium persulfate, or the like as the polymerization initiator, polymerization may be conducted by heating at a temperature of 30°C to 100°C for 1 to 48 hours in a sealed container under an inert atmosphere. In a case of using hydrogen peroxide-ferrous chloride, sodium persulfate-sodium acid sulfite, or the like as the polymerization initiator, polymerization may be conducted at a temperature of 0 to 30°C for 1 to 48 hours in a sealed container under an inert atmosphere.

It should be noted that, after the polymerization, the content is taken out and subjected to Soxhlet extraction with a solvent such as isopropanol to remove the unreacted monomer and the residual surfactant, and thus the monolith A shown in the middle figure of FIG. 2 is obtained.

(Hydrolysis Step) Subsequently, a step (hydrolysis step) of hydrolyzing the monolith A (crosslinked polymer) to obtain the absorbent A will be described.

First, the monolith A is immersed in dichloroethane to which zinc bromide is added, stirred at 40°C for 24 hours, then brought into contact with methanol, 4% hydrochloric acid, a 4% aqueous solution of sodium hydroxide, and water in this order for hydrolysis, and then dried. Thus, a block-shaped absorbent A is obtained. Further, the block-shaped absorbent A is pulverized to a predetermined size to obtain a particulate absorbent A. It should be noted that the form of the absorbent A is not limited to a particulate form, and, for example, the absorbent A may be shaped into a sheet shape while or after drying.

Further, the method for hydrolyzing the monolith A is not particularly limited, and various methods can be adopted. For example, there is a method of bringing a solvent into contact with a strong base such as sodium hydroxide, or a method of bringing a solvent into contact with a hydrohalic acid such as hydrochloric acid, a Brönsted acid such as sulfuric acid, nitric acid, trifluoroacetic acid, methanesulfonic acid, or p-toluenesulfonic acid, or a Lewis acid such as zinc bromide, aluminum chloride, aluminum bromide, titanium (IV) chloride, cerium chloride/sodium iodide, or magnesium iodide using any of various solvent including aromatic solvents such as toluene and xylene, halogen-based solvents such as chloroform and dichloroethane, ether-based solvents such as tetrahydrofuran and isopropyl ether, amide-based solvents such as dimethylformamide and dimethylacetamide, alcohol-based solvents such as methanol and ethanol, carboxylic acid-based solvents such as acetic acid and propionic acid, or water as the solvent.

Further, among the polymerization raw materials of the organic polymer that forms the hydrophilic continuous skeleton of the absorbent A, the (meth)acrylic acid ester is not particularly limited, C1 to C10 (that is, containing 1 to 10 carbon atoms) alkyl ester of (meth)acrylic acid is preferable, and C4 (that is, containing 4 carbon atoms) alkyl ester of (meth)acrylic acid is particularly preferable. It should be noted that as a C4 alkyl ester of (meth)acrylic acid, there are provided (meth)acrylic acid t-butyl ester, (meth)acrylic acid n-butyl ester, and (meth)acrylic acid iso-butyl ester.

Further, the monomer used for the crosslinking polymerization includes (meth)acrylic acid ester and divinylbenzene, and additionally, a monomer other than (meth)acrylic acid ester and divinylbenzene. In the latter case, the monomer other than (meth)acrylic acid ester and divinylbenzene styrene is not particularly limited, and for example, there are provided styrene, α-methylstyrene, vinyltoluene, vinylbenzyl chloride, glycidyl (meth)acrylate, 2-ethylhexyl (meth)acrylate, isobutene, butadiene, isoprene, chloroprene, vinyl chloride, vinyl bromide, vinylidene chloride, tetrafluoroethylene, (meth)acrylonitrile, vinyl acetate, ethylene glycol di(meth)acrylate, trimethylol propane tri(meth)acrylate, and the like. It should be noted that, the ratio of the monomer other than (meth)acrylic acid ester and divinylbenzene in all monomers used for the crosslinking polymerization is preferably 0 to 80 mol%, and more preferably 0 to 50 mol%.

Further, the surfactant is not limited to the above-described sorbitan monooleate, and any surfactant that can form a water-in-oil (W/O) emulsion when a monomer for crosslinking polymerization and water are mixed can be used. As such a surfactant, for example, there are provided nonionic surfactants such as sorbitan monolaurate, sorbitan monopalmitate, sorbitan monostearate, sorbitan trioleate, polyoxyethylene group nonylphenyl ether, polyoxyethylene group stearyl ether, and polyoxyethylene group sorbitan monooleate, anionic surfactants such as potassium oleate, sodium dodecylbenzene sulfonate, and dioctyl sodium sulfosuccinate, cationic surfactants such as distearyl dimethyl ammonium chloride, and amphoretic surfactants such as lauryl dimethyl betaine. These surfactants may be used alone or in combination of two or more types.

In addition, a compound that generates radicals by heat and light irradiation is suitably used as the polymerization initiator. The polymerization initiator may be water-soluble or oil-soluble. For example, there are provided azobis (4-methoxy-2,4-dimethylvaleronitrile), azobisisobutyronitrile, azobisdimethylvaleronitrile, azobiscyclohexanenitrile, azobiscyclohexanecarbonitrile, azobis (2-methylpropionamidine) dihydrochloride, benzoyl peroxide, potassium persulfate, ammonium persulfate, hydrogen peroxide-ferrous chloride, sodium persulfate-sodium acid sulfite, and tetramethylthiuram disulfide. However, in some cases, there is a system in which polymerization proceeds with only heating or only light irradiation even when the polymerization initiator is not added. Therefore, in such a system, the addition of the polymerization initiator is not necessary. Examples

Hereinafter, the present invention will be described by showing examples, but the present invention is not limited to these examples.

### (A) Sample

### (a) Pore distribution

The polymer absorbent of the present invention manufactured by the above-described manufacturing method is used as a sample for Examples 1 to 5, and Infinity particle body is prepared as sample for Comparative Examples 1 to 2. However, for the samples of Examples 1 to 5, when forming water-in-oil emulsion in the manufacturing method, the surfactant/monomer ratio (wt%) and the stirring time (min) are changed. Further, it should be noted that the above Infinity particle body is an absorbent manufactured by P&G Corporation and has a structure similar to the polymer absorbent (foamed structure), but, unlike the polymer absorbent, the Infinity particle body does not have a function of absorbing water and expanding. (b) Relationship between the bulk density and the water absorption rate

Regarding the samples of Examples above, the samples having various bulk densities are prepared by changing the conditions of ethanol immersion and drying. Specifically, the dried polymer absorbent body is sufficiently immersed in a 40% ethanol aqueous solution, then the supernatant liquid is removed, and ethanol is added. By repeating this series of steps, the aqueous solution is adjusted to an ethanol aqueous solution having a predetermined concentration, and the polymer absorbent body is sufficiently immersed. Thereafter, the polymer absorbent body in a wet state is filtered and dried under reduced pressure at 50°C overnight to obtain polymer absorbent bodies having different bulk densities.

### (B) Evaluation

### (a) Pore distribution

For each sample, the cumulative (integrated) pore volume distribution (the relationship between the pore radius and the cumulative pore volume) and the log differential pore volume distribution (the relationship between the pore radius and the log differential pore volume) are obtained by mercury intrusion method, and the total pore volume (mL/g), the average pore radius (µm), the maximum pore radius (µm), and (the ratio of) the pore volume (mL/g) of pores having a predetermined pore radius or more (or less), the pore volume variation coefficient, and the like are calculated. (b) Relationship between the bulk density and the water absorption rate

The relationship between the bulk density and the water absorption rate (DW) of each sample is investigated by measuring the water absorption rate (DW).

### (C) Evaluation results

### (a) Pore distribution

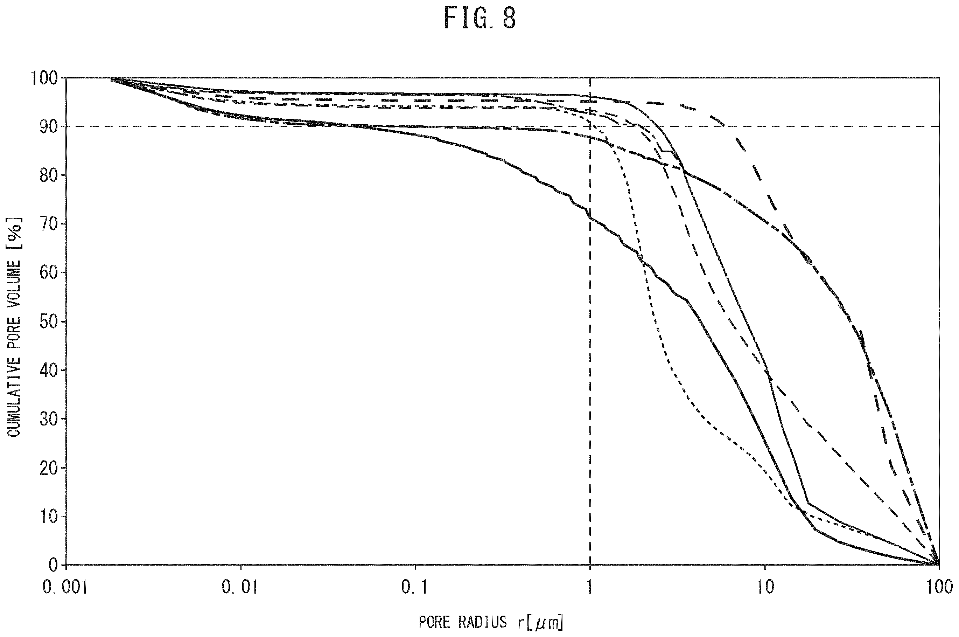

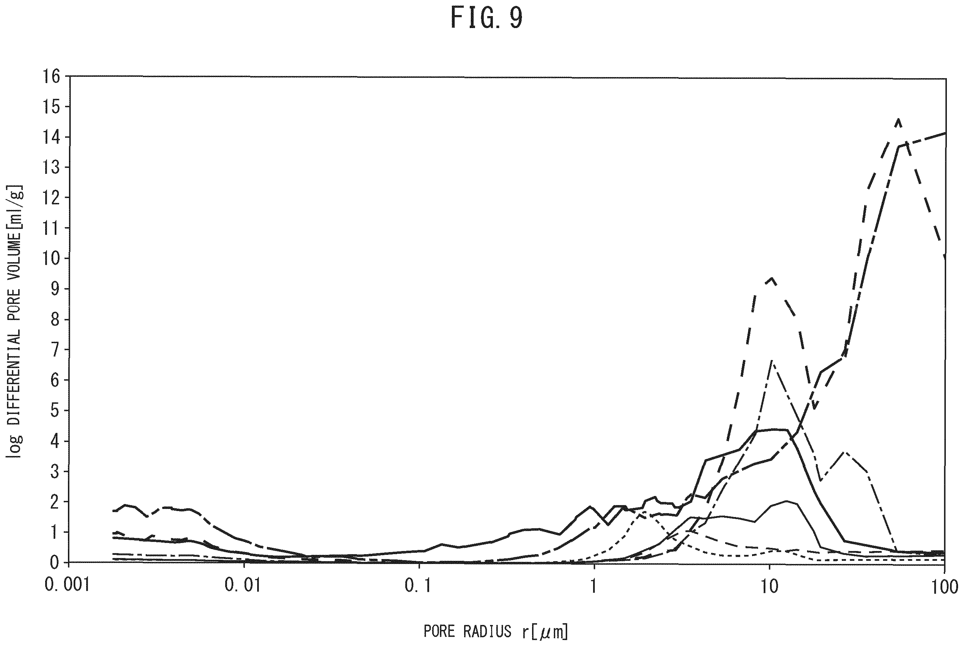

The measurement results are shown in FIGS. 8 and 9, and the summarized contents thereof are shown in Table 1. Here, FIG. 8 shows the obtained cumulative (integrated) pore volume distribution, that is, the relationship between the pore radius (horizontal axis) and the cumulative pore volume (longitudinal axis), and FIG. 9 shows the obtained log differential pore volume distribution, that is, the relationship between the pore radius (horizontal axis) and the log differential pore volume (longitudinal axis). It should be noted that, in FIGS. 8 and 9, a dashed line (thick line) indicates Example 1, a single-dotted chain line (thin line) indicates Example 2, a single-dotted chain line (thin line) indicates Example 3, a dotted line indicates Example 4, a solid line (thin line) indicates Example 5, a solid line (thick line) indicates Comparative Example 1, and a single-dotted chain line (thick line) indicates Comparative Example 2.

**[Table 1]**

| Sample | Surfact ant/mo nomer (wt%) | Stirring time (min) | Total pore volume (mL/g) | Average pore radius (µm) | Maximum pore radius (µm) | Pore volume* (mL/g) | Ratio of pore volume* (%) | Variation coefficient of pore volume |
|---|---|---|---|---|---|---|---|---|
| Example 1 | 10.6 | 2 | 13.5 | 0.086 | 49.1 | 12.8 | 95.2% | 1.8 |
| Example 2 | 5.0 | 55 | 4.43 | 0.096 | 10.0 | 4.2 | 95.9% | 1.94 |
| Example 3 | 7.5 | 5 | 1.07 | 0.062 | 3.5 | 1.0 | 93.4% | 1.38 |
| Example 4 | 7.5 | 10 | 1.77 | 0.110 | 12.9 | 1.7 | 96.2% | 1.56 |
| Example 5 | 7.5 | 30 | 0.94 | 0.071 | 1.9 | 0.9 | 91.4% | 1.65 |
| Comparative Example 1 | - | - | 5.88 | 0.040 | 11.3 | 4.2 | 71.4% | 0.85 |
| Comparative Example 2 | - | - | 13 | 0.039 | 100.0 | 11.4 | 87.9% | 1.26 |

| | | | | | | | | |
|---|---|---|---|---|---|---|---|---|
| * Pore radius of 1 µm or more | | | | | | | | |

In the samples of Examples 1 to 5, the following facts can be confirmed. The ratio of the pore volume of pores having a pore radius of 1 µm or more is 90% or more of the pore volume of all pores. The ratio of the pore volume of the pores having a pore radius of 0.005 µm or less is less than 10% of the pore volume of all of the pores. The pore radius at the maximum value of the pore volume is 500 µm or less. There is a case where the variation coefficient of the pore distribution in the pores having a pore radius of 1 µm or more is 1.4 or less (Example 3). In the pores having a pore radius of 1 µm or more, there are cases where the variation coefficient of the pore distribution is more than 1.4 (Examples 4 and 5). There is a case where, with respect to the pore radius corresponding to the local maximum value of the pore volume in the curved line showing the pore distribution, a portion on the side with a larger pore radius is broader than a portion on the side with a smaller pore radius (Example 5). There is a case where, with respect to the pore radius corresponding to the local maximum value of the pore volume in the curved line showing the pore distribution, a portion on the side with a smaller pore radius is broader than a portion on the side with a larger pore radius (Example 4). There are cases where at least two local maximum values of the pore volume are present in the curved line showing the pore distribution (Examples 1 and 2). There is a case where, of the two local maximum values of the pore volume in the curved line showing the pore distribution, a relatively small local maximum value of the pore radius is larger than a relatively large local maximum value of the pore radius (Example 2). There is a case where, of the two local maximum values of the pore volume in the curved line showing the pore distribution, a relatively small local maximum value of the pore radius is smaller than a relatively large local maximum value of the pore radius (Example 1). The total pore volume is 0.9 mL/g or more. On the other hand, in Comparative Examples 1 and 2, at least the ratio of the pore volume of the pores having a pore radius of 1 µm or more is less than 90% of the pore volume of all of the pores.

### (b) Relationship between the bulk density and the water absorption rate

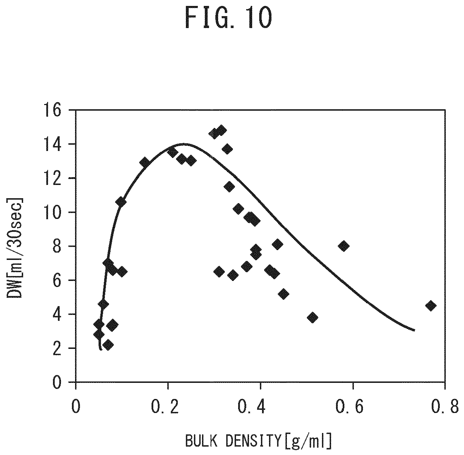

The measurement results are shown in FIG. 10. FIG. 10 is a graph showing the relationship between the obtained bulk density (horizontal axis) and the water absorption rate (longitudinal axis). As shown in the drawing, it can be confirmed that when the bulk density is 0.07 to 0.6 g/cm³, the water absorption rate (DW) performance can be set to 6 mL/30 sec. or more. Further, it can be confirmed that when the bulk density is 0.1 to 0.4 g/cm³, the water absorption rate (DW) performance can be set to 10 mL/30 sec. or more. Further, it can be confirmed that when the bulk density is 0.15 to 0.35 g/cm³, the water absorption rate (DW) performance can be set to 12 mL/30 sec. or more.

It should be noted that, in addition to the light incontinence pad of the above-described embodiment, the composite absorbent body of the present invention can be applied to various sanitary products such as underpants-shaped disposable diapers, tape-type disposable diapers, sanitary napkins, absorbent panty liners, absorbent pads (for example, bedsore pads, puerperal pads and the like), absorbent sheets, breast pads, disposable diapers for pets, absorbent pads for pets, excrement disposal sheets for pets, wet sheets, wet tissues, cosmetic wiping sheets, masks, and the like. Therefore, the bodily fluid that is the liquid to be absorbed of the composite absorbent body is a liquid discharged from a wearer of a sanitary product, and for example, there are provided urine, sweat, feces, menstrual blood, vaginal discharge, breast milk, blood, exudate, and the like.

In addition, the present invention is not limited to the above-described embodiments and the like, and appropriate combinations, substitutions, modifications, and the like can be made without departing from the aspect, gist, and the like of the present invention.

### REFERENCE SIGNS LIST

1: light incontinence pad
2: top sheet
3: back sheet
4: composite absorbent body

## Claims

1. A composite absorbent body (4) for a sanitary product for absorbing a bodily fluid, the composite absorbent body comprising:
a polymer absorbent having a hydrophilic continuous skeleton and a continuous pore, wherein
in the polymer absorbent, a ratio of a pore volume of a pore having a pore radius of 1 µm or more is 90% or more of a pore volume of the pore,
the polymer absorbent is a hydrolysate of the crosslinked polymer of monomers including a (meth) acrylic acid ester and divinylbenzene which is a compound containing two or more vinyl groups in one molecule, and
a ratio of crosslinked polymerization residue of the divinylbenzene in an organic polymer that forms the hydrophilic continuous skeleton is 0.1 to 30 mol % with respect to all of constituent units.

2. The composite absorbent body according to Claim 1, wherein
in the polymer absorbent, a ratio of a pore volume of the pore having the pore radius of 0.005 µm or less is less than 10% of the pore volume of the pore.

3. The composite absorbent body according to Claim 1 or 2, wherein
in the polymer absorbent, a pore radius at a maximum value of a pore volume value is 500 µm or less.

4. The composite absorbent body according to any one of Claims 1 to 3, wherein
in the polymer absorbent, a variation coefficient of a pore distribution in the pores having a pore radius of 1 µm or more is 1.4 or less.

5. The composite absorbent body according to any one of Claims 1 to 3, wherein
in the polymer absorbent, a variation coefficient of a pore distribution in the pores having a pore radius of 1 µm or more is more than 1.4.

6. The composite absorbent body according to Claim 5, wherein
in the polymer absorbent, a portion on a side with a larger pore radius is broader than a portion on a side with a smaller pore radius with respect to a pore radius corresponding to a local maximum value of the pore volume in a curved line showing a pore distribution.

7. The composite absorbent body according to Claim 5, wherein
in the polymer absorbent, a portion on a side with a smaller pore radius is broader than a portion on a side with a larger pore radius with respect to a pore radius corresponding to a local maximum value of the pore volume in a curved line showing a pore distribution.

8. The composite absorbent body according to any one of Claims 1 to 3, wherein
in the polymer absorbent, at least two local maximum values of the pore volume are present in a curved line showing a pore distribution.

9. The composite absorbent body according to Claim 8, wherein
in the polymer absorbent, of the two local maximum values of the pore volume in the curved line showing the pore distribution, a relatively small local maximum value of a pore radius is larger than a relatively large local maximum value of the pore radius.

10. The composite absorbent body according to Claim 8, wherein
in the polymer absorbent, of the two local maximum values of the pore volume in the curved line showing the pore distribution, a relatively small local maximum value of a pore radius is smaller than a relatively large local maximum value of the pore radius.

11. The composite absorbent body according to any one of Claims 1 to 10, wherein
the polymer absorbent has a total pore volume of 0.9 mL/g or more.

12. The composite absorbent body according to any one of Claims 1 to 11, wherein
the polymer absorbent has a bulk density of 0.07 to 0.6 g/cm3.

13. The composite absorbent body according to any one of Claims 1 to 12, wherein
the polymer absorbent is a monolithic absorbent.

14. The composite absorbent body according to any one of Claims 1 to 13, wherein
the polymer absorbent is a hydrolysate of a crosslinked polymer of a (meth)acrylic acid ester and a compound containing two or more vinyl groups in one molecule, and contains at least one or more -COONa groups.

15. A sanitary product (1) comprising:
the composite absorbent body according to any one of Claims 1 to 14.

## Patentansprüche

1. Zusammengesetzter Saugkörper (4) für ein Hygieneprodukt zur Absorption einer Körperflüssigkeit, wobei der zusammengesetzte Saugkörper Folgendes umfasst:
ein Polymerabsorptionsmittel, das ein hydrophiles kontinuierliches Gerüst und eine kontinuierliche Pore aufweist, wobei
in dem Polymerabsorptionsmittel ein Verhältnis eines Porenvolumens einer Pore, die einen Porenradius von 1 µm oder mehr aufweist, 90 % oder mehr eines Porenvolumens der Pore beträgt,
das Polymerabsorptionsmittel ein Hydrolysat des vernetzten Polymers von Monomeren ist, die einen (Meth)acrylsäureester und Divinylbenzol, das eine Verbindung ist, die zwei oder mehr Vinylgruppen in einem Molekül enthält, einschließt, und
ein Verhältnis von vernetztem Polymerisationsrest des Divinylbenzols in einem organischen Polymer, das das hydrophile kontinuierliche Gerüst bildet, 0,1 bis 30 mol-% in Bezug auf alle Bestandteileinheiten beträgt.

2. Zusammengesetzter Saugkörper nach Anspruch 1, wobei
in dem Polymerabsorptionsmittel ein Verhältnis eines Porenvolumens der Pore, die den Porenradius von 0,005 µm oder kleiner aufweist, weniger als 10 % des Porenvolumens der Pore beträgt.

3. Zusammengesetzter Saugkörper nach Anspruch 1 oder 2, wobei
in dem Polymerabsorptionsmittel ein Porenradius bei einem maximalen Wert eines Porenvolumens 500 µm oder weniger beträgt.

4. Zusammengesetzter Saugkörper nach einem der Ansprüche 1 bis 3, wobei
in dem Polymerabsorptionsmittel ein Variationskoeffizient einer Porenverteilung in den Poren, die einen Porenradius von 1 µm oder größer aufweisen, 1,4 oder weniger beträgt.

5. Zusammengesetzter Saugkörper nach einem der Ansprüche 1 bis 3, wobei
in dem Polymerabsorptionsmittel ein Variationskoeffizient einer Porenverteilung in den Poren, die einen Porenradius von 1 µm oder größer aufweisen, mehr als 1,4 beträgt.

6. Zusammengesetzter Saugkörper nach Anspruch 5, wobei
in dem Polymerabsorptionsmittel ein Teil auf einer Seite mit einem größeren Porenradius breiter ist als ein Teil auf einer Seite mit einem kleineren Porenradius, in Bezug auf einen Porenradius, der einem lokalen maximalen Wert des Porenvolumens in einer gekrümmten Linie, die eine Porenverteilung zeigt, entspricht.

7. Zusammengesetzter Saugkörper nach Anspruch 5, wobei
in dem Polymerabsorptionsmittel ein Teil auf einer Seite mit einem kleineren Porenradius breiter ist als ein Teil auf einer Seite mit einem größeren Porenradius, in Bezug auf einen Porenradius, der einem lokalen maximalen Wert des Porenvolumens in einer gekrümmten Linie, die eine Porenverteilung zeigt, entspricht.

8. Zusammengesetzter Saugkörper nach einem der Ansprüche 1 bis 3, wobei
in dem Polymerabsorptionsmittel mindestens zwei lokale maximale Werte des Porenvolumens in einer gekrümmten Linie, die eine Porenverteilung zeigt, vorliegen.

9. Zusammengesetzter Saugkörper nach Anspruch 8, wobei
in dem Polymerabsorptionsmittel von den zwei lokalen maximalen Werten des Porenvolumens in der gekrümmten Linie, die die Porenverteilung zeigt, ein relativ kleiner lokaler maximaler Wert eines Porenradius größer ist als ein relativ großer lokaler maximaler Wert des Porenradius.

10. Zusammengesetzter Saugkörper nach Anspruch 8, wobei
in dem Polymerabsorptionsmittel von den zwei lokalen maximalen Werten des Porenvolumens in der gekrümmten Linie, die die Porenverteilung zeigt, ein relativ kleiner lokaler maximaler Wert eines Porenradius kleiner ist als ein relativ großer lokaler maximaler Wert des Porenradius.

11. Zusammengesetzter Saugkörper nach einem der Ansprüche 1 bis 10, wobei
das Polymerabsorptionsmittel ein gesamtes Porenvolumen von 0,9 ml/g oder mehr aufweist.

12. Zusammengesetzter Saugkörper nach einem der Ansprüche 1 bis 11, wobei
das Polymerabsorptionsmittel eine Schüttdichte von 0,07 bis 0,6 g/cm3 aufweist.

13. Zusammengesetzter Saugkörper nach einem der Ansprüche 1 bis 12, wobei
das Polymerabsorptionsmittel ein monolithisches Absorptionsmittel ist.

14. Zusammengesetzter Saugkörper nach einem der Ansprüche 1 bis 13, wobei
das Polymerabsorptionsmittel ein Hydrolysat eines vernetzten Polymers eines (Meth)acrylsäureesters und einer Verbindung, die zwei oder mehr Vinylgruppen in einem Molekül enthält, ist und mindestens eine oder mehrere -COONa-Gruppen enthält.

15. Hygieneprodukt (1), das Folgendes umfasst:
den zusammengesetzten Saugkörper nach einem der Ansprüche 1 bis 14.

## Revendications

1. Corps absorbant composite (4) pour un produit hygiénique destiné à absorber un fluide corporel, le corps absorbant composite comportant :
un absorbant polymère ayant un squelette continu hydrophile et un pore continu, dans lequel
dans l'absorbant polymère, un rapport du volume poreux d'un pore ayant un rayon de pore de 1 µm ou plus est de 90 % ou plus d'un volume poreux du pore,
l'absorbant polymère est un hydrolysat d'un polymère réticulé de monomères comprenant un ester d'acide (méth)acrylique et de divinylbenzène qui est un composé contenant deux ou plusieurs groupes vinyle dans une molécule, et
un rapport d'un résidu de polymérisation réticulé de divinylbenzène dans un polymère organique qui forme le squelette continu hydrophile est compris entre 0,1 et 30 % en moles par rapport à toutes les unités constituantes.

2. Corps absorbant composite selon la revendication 1, dans lequel
dans l'absorbant polymère, un rapport d'un volume poreux du pore ayant un rayon de pore inférieur ou égal à 0,005 µm est inférieur à 10 % du volume poreux du pore.

3. Corps absorbant composite selon la revendication 1 ou la revendication 2, dans lequel
dans l'absorbant polymère, un rayon de pore à une valeur maximale d'une valeur du volume poreux est de 500 µm ou moins.

4. Corps absorbant composite selon l'une quelconque des revendications 1 à 3, dans lequel
dans l'absorbant polymère, un coefficient de variation d'une distribution des pores dans les pores ayant un rayon de pore de 1 µm ou plus est de 1,4 ou moins.

5. Corps absorbant composite selon l'une quelconque des revendications 1 à 3, dans lequel
dans l'absorbant polymère, un coefficient de variation d'une distribution des pores dans les pores ayant un rayon de pore de 1 µm ou plus est supérieur à 1,4.

6. Corps absorbant composite selon la revendication 5, dans lequel
dans l'absorbant polymère, une partie sur un côté ayant un rayon de pore plus grand est plus large qu'une partie sur un côté ayant un rayon de pore plus petit par rapport à un rayon de pore correspondant à une valeur maximale locale du volume poreux dans une ligne courbe représentant une distribution de pores.

7. Corps absorbant composite selon la revendication 5, dans lequel
dans l'absorbant polymère, une partie sur un côté ayant un rayon de pore plus petit est plus large qu'une partie sur un côté ayant un rayon de pore plus grand par rapport à un rayon de pore correspondant à une valeur maximale locale du volume poreux dans une ligne courbe représentant une distribution de pores.

8. Corps absorbant composite selon l'une quelconque des revendications 1 à 3, dans lequel
dans l'absorbant polymère, au moins deux valeurs maximales locales du volume poreux sont présentes sur une ligne courbe représentant une distribution des pores.

9. Corps absorbant composite selon la revendication 8, dans lequel
dans l'absorbant polymère, parmi les deux valeurs maximales locales du volume poreux dans la ligne courbe représentant la distribution des pores, une valeur maximale locale relativement petite d'un rayon de pore est supérieure à une valeur maximale locale relativement grande du rayon de pore.

10. Corps absorbant composite selon la revendication 8, dans lequel
dans l'absorbant polymère, parmi les deux valeurs maximales locales du volume poreux dans la ligne courbe représentant la distribution des pores, une valeur maximale locale relativement petite d'un rayon de pore est inférieure à une valeur maximale locale relativement grande du rayon de pore.

11. Corps absorbant composite selon l'une quelconque des revendications 1 à 10, dans lequel l'absorbant polymère a un volume poreux total de 0,9 mL/g ou plus.

12. Corps absorbant composite selon l'une quelconque des revendications 1 à 11, dans lequel l'absorbant polymère a une masse volumique apparente comprise entre 0,07 et 0,6 g/cm3.

13. Corps absorbant composite selon l'une quelconque des revendications 1 à 12, dans lequel l'absorbant polymère est un absorbant monolithique.

14. Corps absorbant composite selon l'une quelconque des revendications 1 à 13, dans lequel l'absorbant polymère est un hydrolysat d'un polymère réticulé d'un ester d'acide (méth)acrylique et de divinylbenzène qui est un composé contenant deux ou plusieurs groupes vinyle dans une molécule, et contient au moins un ou plusieurs groupes -COONa.

15. Produit hygiénique (1) comportant :
le corps absorbant composite selon l'une quelconque des revendications 1 à 14.
